(19) Europäisches Patentamt
European Patent Office
Office européen des brevets

(11) EP 1 312 338 B1

(12) FASCICULE DE BREVET EUROPEEN

(45) Date de publication et mention de la délivrance du brevet:
**08.09.2010 Bulletin 2010/36**

(51) Int Cl.:
***A61K 8/898*** *(2006.01)* ***A61Q 5/04*** *(2006.01)*

(21) Numéro de dépôt: **02292783.4**

(22) Date de dépôt: **07.11.2002**

(54) **Procédé de déformation permanente des cheveux mettant en oeuvre des silicones aminées particulières**

Verfahren zur dauerhaften Haarverformung unter Anwendung von speziellen Aminosilikonen

Permanent hair-waving process using particular aminosilicones

(84) Etats contractants désignés:
**AT BE BG CH CY CZ DE DK EE ES FI FR GB GR IE IT LI LU MC NL PT SE SK TR**

(30) Priorité: **08.11.2001 FR 0114478**

(43) Date de publication de la demande:
**21.05.2003 Bulletin 2003/21**

(73) Titulaire: **L'Oréal**
**75008 Paris (FR)**

(72) Inventeurs:
- **Devin-Baudoin, Priscille**
  **92170 Vanves (FR)**
- **Sabbagh, Anne**
  **92500 Rueil Malmaison (FR)**

(74) Mandataire: **Bourdeau, Françoise**
**L'Oréal**
**D.I.P.I.**
**25-29 Quai Aulagnier**
**92600 Asnieres (FR)**

(56) Documents cités:
**GB-A- 2 141 454 US-A- 5 340 367**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de déformation permanente des fibres kératiniques, en particulier des cheveux, ledit procédé étant notamment utilisable dans les salons de coiffure, professionnels ou par des particuliers, via la commercialisation de kits.

Au sens de la présente invention, on entend par «*procédé de déformation permanente*», tout procédé durable dans le temps, de mise en forme des cheveux, de frisage, de défrisage ou de décrêpage.

On entend par «*fibres kératiniques*», en particulier, les cheveux, les cils, les sourcils, et surtout les cheveux.

On sait que la technique la plus usuelle pour obtenir une déformation permanente des cheveux consiste, dans un premier temps, à réaliser l'ouverture des liaisons disulfures - S-S- de la kératine (cystine) à l'aide d'une composition réductrice, contenant un agent réducteur (étape de réduction) puis, après avoir de préférence, rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème des techniques de permanentes connues à ce jour est que leur application répétée sur les cheveux induit à la longue une altération progressive de la qualité du cheveu, et notamment une altération progressive et marquée de la brillance et des propriétés cosmétiques du cheveu, en particulier au niveau de la douceur des fibres qui ont tendance à devenir de plus en plus rêches ainsi qu'au niveau de leur démêlage, les cheveux devenant de plus en plus difficiles à démêler. Cette altération est en outre particulièrement accentuée lorsque l'étape de fixation de l'opération de déformation permanente est effectuée à l'aide d'un bromate.

Pour limiter cette altération des cheveux, il a déjà été proposé d'introduire directement, dans la composition réductrice, des agents de conditionnement. Par exemple, les demandes de brevet japonais H2-250814 et H9-151120 décrivent des compositions réductrices contenant des silicones aminées, pouvant éventuellement se présenter sous la forme de microémulsion.

Le document GB 2 141 454 décrit des compositions conditionnantes pour les cheveux comprenants un agent oxydant et une silicone aminée sous forme émulsionnée.

Cependant, les procédés de déformation permanente des cheveux utilisant de telles compositions ne donnent pas encore entière satisfaction, dans la mesure où le degré, la qualité et la nervosité de frisure sont généralement insuffisants et éphémères, comme si l'agent de conditionnement, notamment les silicones aminées, directement associé à l'agent réducteur, freinaient l'activité de ce dernier.

Le problème posé par l'invention est de fournir un procédé de déformation permanente des fibres kératiniques, notamment des cheveux, qui réduise la dégradation mécanique et/ou cosmétique des cheveux, tout en apportant un degré, une qualité et une nervosité de frisure satisfaisants.

La Demanderesse a découvert, de manière surprenante et inattendue, qu'en appliquant sur les cheveux, avant d'appliquer la composition réductrice et/ou après avoir appliqué la composition oxydante, au moins une composition cosmétique de pré-traitement et/ou de post-traitement contenant au moins une silicone aminée bien particulière, il était possible de résoudre le problème posé par la présente invention.

L'invention a pour objet un procédé de déformation permanente des fibres kératiniques, notamment des cheveux, comprenant au moins les opérations consistant à :

(i) appliquer sur les fibres kératiniques une composition réductrice ;
(ii)réaliser l'oxydation des fibres kératiniques,

caractérisé par le fait qu'il comprend, en outre, l'application sur les fibres kératiniques, avant l'opération (i) et/ou après l'opération (ii), d'une composition cosmétique de pré-traitement et/ou de post-traitement comprenant, dans un véhicule cosmétiquement acceptable, au moins une silicone aminée sous forme non microémulsionnée, choisie parmi les silicones aminées de formule (I) ou (II) suivantes :

Silicones aminées de formule (I)

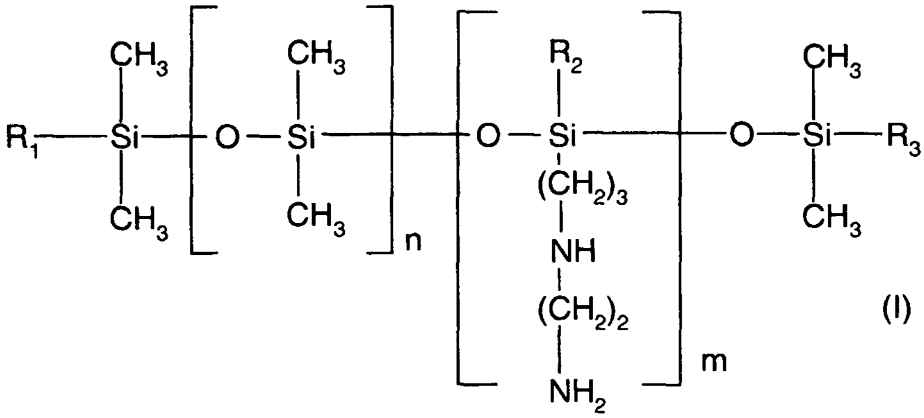

(I)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5;

$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.

Le rapport molaire Hydroxy/Alcoxy est, de préférence, compris entre 0,2:1 et 0,4:1 et de préférence entre 0,25:1 et 0,35:1 et plus particulièrement est égal à 0,3.

La silicone aminée de formule (I) présente une masse moléculaire moyenne en poids allant, de préférence, de 2000 à 1000000 et encore plus particulièrement, de 3500 à 200000.

Silicones aminées de formule (II)

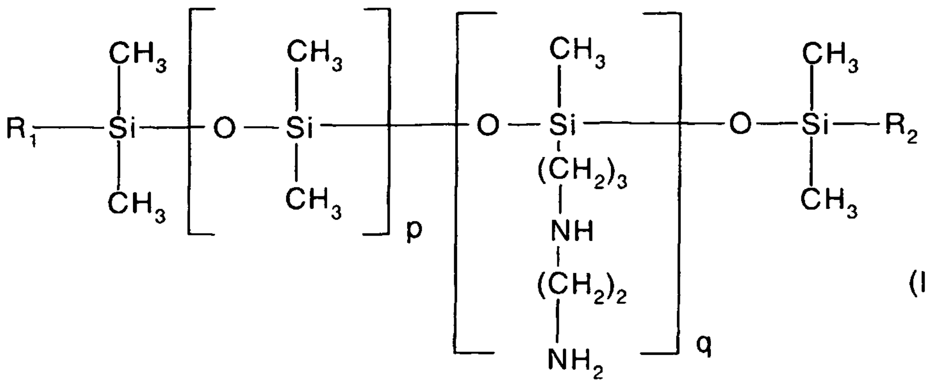

(II)

dans laquelle :

p et q sont des nombres tels que la somme (p + q) peut varier notamment de 1 à 1 000 et en particulier de 50 à 350, et plus particulièrement de 150 à 250
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;

$R_1$, $R_2$, différents, représentent un radical hydroxy ou alcoxy en $C_1$-$C_4$, l'un au moins des radicaux $R_1$ à $R_2$ désignant un radical alcoxy.

De préférence le radical alcoxy est un radical méthoxy.
Le rapport molaire Hydroxy/Alcoxy est de préférence compris entre 1:0,8 et 1:1,1 et de préférence entre 1:0,9 et 1:1 et plus particulièrement est égal à 1:0,95.
La silicone aminée de formule (II) présente une masse moléculaire moyenne en poids allant de préférence de 2000 à 200.000, et encore plus particulièrement de 5.000 à 100.000 et plus particulièrement encore de 10.000 à 50.000.
Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.
Par non microémulsionnée on entend au sens de la présente invention une silicone utilisée tel quel ou sous forme d'une émulsion en présence d'un ou plusieurs tensioactifs, la taille moyenne en nombre des particules de silicone dans l'émulsion étant supérieure à 70 nm et de préférence comprise entre 70 et 500 nm. Les tensioactifs peuvent être de toute nature mais de préférence cationique et/ou non ionique.
Un autre objet de l'invention concerne un kit pour la déformation permanente des fibres kératiniques, l'un des compartiments contenant une composition cosmétique de pré-traitement et/ou de post-traitement comprenant au moins une silicone aminée telle que définie ci-dessus.
Les produits commerciaux correspondant à ces silicones de structure (I) ou (II) peuvent inclure dans leur composition une ou plusieurs autres silicones aminées dont la structure est différente des structures (I) et (II).
Un produit contenant des silicones aminées de structure (I) est proposé par la Société WACKER sous la dénomination BELSIL ADM 652 ®.
Un produit contenant des silicones aminées de structure (II) est proposé par la société WACKER sous la dénomination Fluid WR 1300®.
De préférence, la silicone aminée de formule (I) ou (II) est choisie de telle façon que l'angle de contact avec l'eau d'un cheveu traité avec une composition contenant 2% MA (matières actives) de ladite silicone selon l'invention soit compris entre 90 et 180°(bornes incluses) et de préférence entre 90 et 130°(bornes incluses).
De préférence, la composition contenant la ou les silicones aminées de formule (I) ou (II) est telle que l'angle de contact d'un cheveu traité avec la dite composition est compris entre 90 et 180°(bornes incluses) et de préférence entre 90 et 130°(bornes incluses).
La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est compris entre 0 et 90°, hydrophobe lorsque cet angle est compris entre 90° et 180°.
Le test s'effectue avec des mèches de cheveux naturels ayant été décolorés dans les mêmes conditions puis lavés.
Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée pendant 30 secondes. La mèche essorée est laissée à l'air libre jusqu'à ce qu'elle soit complètement sèche.
Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA («Dynamic Contact Angle Analyser »), de la société CAHN Instruments, permet de mesurer la force (F) exercée par l'eau sur le cheveu.
Parallèlement, le périmètre du cheveu (P) est mesuré *via* une observation microscopique.
La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, $\Gamma lv$ la tension interfaciale liquide / vapeur de l'eau en $J/m^2$ et $\theta$ l'angle de contact.

Le produit SLM 28020® de WACKER à 12% dans l'eau (soit 2% enmatières actives)) conduit à un angle de contact de 93° selon le test indiqué ci-dessus.

Avantageusement, la concentration en silicone aminée dans la composition de pré-traitement ou de post-traitement est comprise entre 0,05 et 10 % en poids par rapport au poids total de cette composition, et plus avantageusement entre 0,1,et 7 %.

Les compositions de pré-traitement ou de post-traitement contenant au moins une silicone aminée utilisées selon l'invention peuvent contenir des actifs hydrosolubles ou liposolubles, ayant une activité cosmétique ou dermopharmaceutique. On peut citer, à titre d'exemples d'actif, les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes, des solvants et des parfums.

Elles peuvent aussi comprendre des solvants et en particulier des alcools inférieurs en C1C8 tels que l'éthanol.

De préférence, le pH de la composition de pré-traitement ou de post-traitement comprenant au moins une silicone aminée est compris de préférence entre 2 et 10, et plus préférentiellement entre 3 et 9.

La composition de pré-traitement comprenant au moins une silicone aminée telle que définie ci dessusest appliquée sur les cheveux à traiter, lesquels auront été, éventuellement, préalablement mouillés. Cette application peut être réalisée après l'habituelle étape de mise sous tension des cheveux sous une forme correspondant à la forme finale désirée pour ces derniers (boucles par exemple), cette étape pouvant, elle-même, être mise en oeuvre par tout moyen, mécanique notamment, approprié et connu en soi pour maintenir sous tension des cheveux, tels que par exemple rouleaux, bigoudis et analogues.

Avantageusement, on laisse agir, à température ambiante ou sous chaleur, sur les cheveux la composition de pré-traitement ou de post-traitement comprenant au moins une silicone aminée pendant une durée comprise entre 1 et 60 minutes, et plus avantageusement entre 3 et 30 minutes.

Selon une étape facultative du procédé, on peut rincer les cheveux imprégnés de la composition de pré-traitement comprenant au moins une silicone aminée, le rinçage étant effectué généralement à l'eau.

Dans une étape indispensable du procédé selon l'invention, on applique sur les cheveux une composition réductrice, ladite composition réductrice contenant généralement au moins un thiol.

Le thiol de la composition réductrice peut être choisi parmi les thiols connus comme agents réducteurs tels que par exemple l'acide thioglycolique, le monothioglycolate de glycérol ou de glycol, la cystéamine et ses dérivés acylés en $C_1$-$C_4$ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide 3-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)$\omega$-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448.

On utilise de préférence l'acide thioglycolique, l'acide thiolactique et l'acide 3-mercaptopropionique.

Les agents réducteurs sont généralement présents à une concentration qui peut être comprise entre 1 et 20 % en poids par rapport au poids total de la compositon réductrice.

Le pH de la composition réductrice est, généralement, compris entre 6 et 10 et de préférence entre 7 et 9.

Les pH des compositions réductrices peuvent être ajustés clasiquement par ajout d'agents basifiants, tels que par exemple l'ammoniaque, la monoéthanolamine, la diéthanolamine, la triéthanolamine, l'isopropanolamine, la propanediamine-1,3, un carbonate ou bicarbonate de métal alcalin ou d'ammonium, un carbonate ou bicarbonate d'amines primaires, secondaires ou tertiaires ou un carbonate organique tel que le carbonate de guanidine, tous ces composés pouvant bien entendu être pris seuls ou en mélange.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol ou de l'isopropanol ou encore du glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Il convient, de manière classique, de laisser reposer pendant quelques minutes, généralement entre 2 et 40 minutes, de préférence entre 5 et 30 minutes, la chevelure sur laquelle a été appliquée la composition réductrice, et ceci de façon à bien laisser le temps au réducteur d'agir correctement sur les cheveux. Cette phase d'attente est effectuée généralement en laissant reposer à l'air libre (à température ambiante ou avec chauffage) la chevelure traitée. Pendant cette phase d'attente, on prend généralement soin que les cheveux ne sèchent pas complètement et restent humides.

Les cheveux imprégnés de la composition réductrice peuvent être, ensuite, rincés soigneusement, généralement à l'eau. Eventuellement, après rinçage, on met en oeuvre une phase de chauffage à température élevée pendant quelques secondes.

Puis, on applique sur les cheveux ainsi rincés une composition oxydante dans le but de fixer la nouvelle forme imposée aux cheveux. Il peut également être envisagé de laisser les cheveux s'oxyder à l'air.

La composition oxydante contient un agent oxydant qui peut être choisi parmi l'eau oxygénée, un bromate alcalin, un persel ou un polythionate. Comme indiqué précédemment, l'un des grands avantages du procédé selon l'invention est qu'il convient parfaitement dans le cas des compositions oxydantes à base de bromates. La concentration en bromates dans la composition oxydante est généralement comprise entre 0,1 et 2 M.

Le pH de la composition oxydante est généralement compris entre 2 et 10.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

La composition de post-traitement comprenant au moins une silicone aminée telle que définie ci-dessus est, de préférence, appliquée après rinçage de la composition oxydante, sur des cheveux humides ou secs. Les cheveux ayant subi le post-traitement peuvent éventuellement être séchés et/ou chauffées et/ou rincés, avant le coiffage. Le cas échéant, la composition peut être appliquée alors que les cheveux sont maintenus par un dispositif mécanique, comme des rouleaux de mise en pli ou des bigoudis.

Le plus souvent, les cheveux imprégnés de la composition oxydante sont rincés soigneusement, généralement à l'eau. On sépare, avant ou après le rinçage, la fibre kératinique des moyens nécessaire à la mise sous tension.

On obtient finalement une chevelure présentant de bonnes propriétés cosmétiques : les cheveux sont plus brillants, plus doux et plus faciles à démêler ou à coiffer.

De manière avantageuse, la composition de pré-traitement ou de post-traitement contenant au moins une silicone aminée telle que définie ci-dessus est appliquée selon l'une au moins des variantes suivantes :

- sur des cheveux propres et humides, avant la mise en oeuvre des moyens de mise sous tension des cheveux, sans rincer les cheveux avant l'application du réducteur ;
- sur des cheveux humides après rinçage du fixateur, les cheveux étant ultérieurement soit rincés, soit séchés.

Lorsque le procédé de déformation permanente des cheveux est un procédé de défrisage, on peut, de manière connue en soi, mettre en oeuvre, un défrisant généralement soit thiolé, soit alcalin.

Dans le cas de défrisant thiolé, le procédé conforme à l'invention est avantageusement mis en oeuvre en appliquant la composition de pré-traitement ou de post-traitement contenant au moins une silicone aminée telle que définie ci-dessus selon l'une au moins des variantes suivantes :

- sur des cheveux propres et humides, sans rinçage avant l'application du réducteur ;
- sur des cheveux propres et humides, après le rinçage du fixateur, en rinçant avant le séchage des cheveux.

Dans le cas de défrisant alcalin, le procédé conforme à l'invention est avantageusement mis en oeuvre en appliquant la composition de pré-traitement ou de post-traitement contenant au moins une silicone aminée telle que

définie ci-dessussur cheveux humides, après rinçage de shampooing neutralisant, en rinçant avant le séchage des cheveux.

Dans le cas d'un frisage des cheveux, le procédé conforme à l'invention procure des boucles nerveuses et les cheveux sont spécialement souples, légers, soyeux et bien individualisés.

Dans le cas d'un défrisage des cheveux, le procédé conforme à l'invention procure notamment une maîtrise du volume de la chevelure, un lissage des cheveux de la racine à la pointe et un toucher plus naturel.

L'invention pourra être mieux comprise à l'aide des exemples non limitatifs qui suivent et qui constituent des modes de mise en oeuvre avantageux du procédé selon l'invention.

m.a. signifie matière active.

<u>EXEMPLES</u> :

1) Composition de soin des cheveux défrisés

| Ingrédients | % ma |
|---|---|
| - alcool cétylstéarylique / laurylsulfate de sodium / myristate de cétyle / alcool myristique (62/20/8/10) | 12 |
| - alcool oléique oxyéthyléné (20 OE) | 0.1 |
| - glycérine | 0.5 |
| - Polydiméthylsiloxane de formule (II) proposée par Wacker sous le nom Finish WR 1300 | 2 |
| - eau déminéralisée | qsp 100 g |

Cette composition est appliquée sur cheveux défrisés avec un défrisant alcalin, après le rinçage du shampooing de nettoyage. La composition est laissée pendant 1 à 10 minutes sur la chevelure avant d'être rincée.

2) Composition de soin protecteur avant permanente

La composition décrite en 1) peut s'utiliser en traitement pré-permanente. Cette composition est appliquée sur cheveux humides avant la pose des bigoudis, et n'est pas rincée avant l'application du réducteur de permanente. Cette composition permet de limiter la dégradation du cheveu due à la permanente, tout en permettant d'obtenir des boucles toniques.

Des résultats analogues sont obtenus en remplaçant le polydiméthylsiloxane de formule (II) par une quantité équivalente de polydiméthylsiloxane de formule (I) proposé par Wacker sous le nom BELSIL ADM 652.

## Revendications

1. Procédé de déformation permanente des fibres kératiniques, notamment des cheveux, comprenant au moins les opérations consistant à :

   (i) appliquer sur les fibres kératiniques une composition réductrice ;
   (ii) réaliser l'oxydation des fibres kératiniques,

   **caractérisé par le fait qu'**il comprend, en outre, l'application sur les fibres kératiniques, avant l'opération (i) et/ou après l'opération (ii), d'une composition cosmétique de pré-traitement et/ou de post-traitement comprenant, dans un véhicule cosmétiquement acceptable, au moins une silicone aminée sous forme non microémulsionnée de formules (I) ou (II), suivantes :

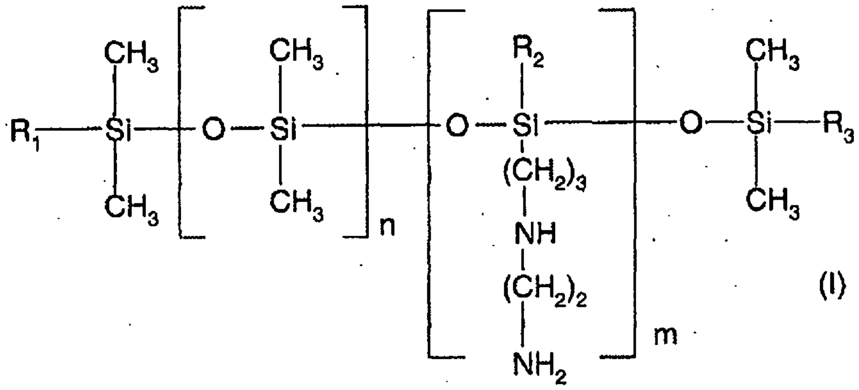

(I)

formule (I) dans laquelle :

m et n sont des nombres tels que la somme (n + m) varie de 1 à 1 000,
n désignant un nombre de 0 à 999
et m désignant un nombre de 1 à 1 000;
$R_1$, $R_2$, $R_3$, identiques ou différents, représentent un radical hydroxy ou alcoxy en $C_1$-$C_4$ ; l'un au moins des radicaux $R_1$ à $R_3$ désignant un radical alcoxy ;

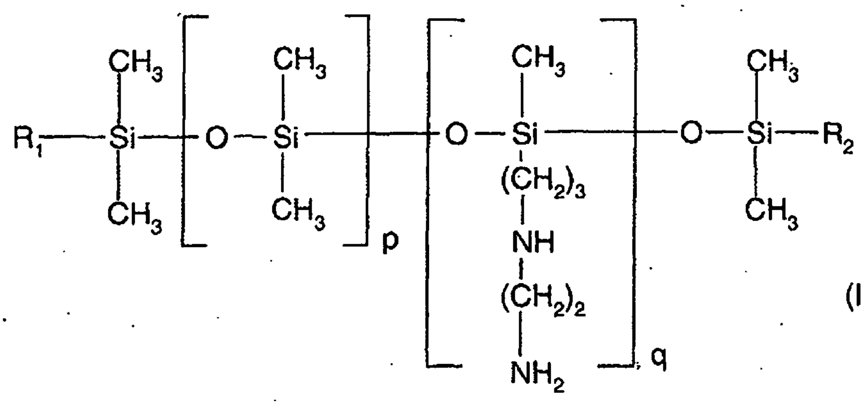

(II)

formule (II) dans laquelle :

p et q sont des nombres tels que la somme (p + q) varie de 1 à 1 000,
p désignant un nombre de 0 à 999 et q désignant un nombre de 1 à 1 000,
$R_1$, $R_2$, différents, représentent un radical hydroxy ou alcoxy en $C_1$-$C_4$, l'un au moins des radicaux $R_1$ à $R_2$ désignant un radical alcoxy.

2. Procédé selon la revendication 1, **caractérisé par le fait que** le radical alkoxy $C_1$-$C_4$ désigne le radical méthoxy.

3. Procédé selon l'une quelconque des revendications précédentes, **caractérisé par le fait que**, pour les silicones aminées de formule (I), le rapport molaire hydroxy/alcoxy est compris entre 0,2:1 et 0,4:1.

4. Procédé selon l'une quelconque des revendications 1 à 2, **caractérisé par le fait que**, pour les silicones aminées de formule (II), le rapport molaire Hydroxy/Alcoxy est compris entre 1:0,8 et 1:1,1.

5. Procédé selon l'une quelconque des revendications 1 à 3, **caractérisé par le fait que** la silicone aminée de formule (I) présente une masse moléculaire moyenne en poids allant de 2000 à 1000000.

6. Procédé selon l'une quelconque des revendications 1 à 2 et 4, **caractérisé par le fait que** la silicone aminée de formule (II) présente une masse moléculaire moyenne en poids allant de 2000 à 200.000.

7. Procédé selon l'une quelconque des revendications précédentes, **caractérisé par le fait que** le pH de la composition de pré-traitement ou de post-traitement comprenant au moins une silicone aminée est compris de préférence entre 2 et 10.

8. Procédé selon l'une quelconque des revendications précédentes, **caractérisé par le fait que** la concentration en silicones aminées non microémulsionnées de formules (I) ou (II) dans la composition de pré-traitement ou de post-traitement est comprise entre 0,05 et 10 % en poids par rapport au poids total de cette composition.

9. Procédé selon l'une quelconque des revendications précédentes, **caractérisé par le fait qu'**on laisse agir sur les cheveux la composition de pré-traitement ou de post-traitement pendant une durée comprise entre 1 et 60 minutes.

10. Procédé selon l'une quelconque des revendications précédentes, **caractérisé par le fait que** la composition de pré-traitement ou de post-traitement comprend, en outre, des additifs choisis parmi les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes, des solvants et des parfums.

11. Kit pour la déformation permanente des fibres kératiniques, l'un des compartiments contenant une composition cosmétique de pré-traitement et/ou de post-traitement comprenant au moins une silicone aminée telle que définie dans l'une quelconque des revendications 1 à 8.

12. Utilisation d'une composition cosmétique de pré-traitement et/ou de post-traitement comprenant, dans un véhicule cosmétiquement acceptable, au moins une silicone aminée telle que définie dans l'une quelconque des revendications 1 à 8, pour la déformation permanente des fibres kératiniques.

## Claims

1. Process for permanently reshaping keratin fibres, especially the hair, comprising at least the operations consisting in:

   (i) applying a reducing composition to the keratin fibres;
   (ii) oxidizing the keratin fibres, **characterized in that** it also involves applying to the keratin fibres, before operation (i) and/or after operation (ii), a pre-treatment and/or post-treatment cosmetic composition comprising, in a cosmetically acceptable vehicle, at least one aminosilicone in non-microemulsified form of formula (I) or (II) below:

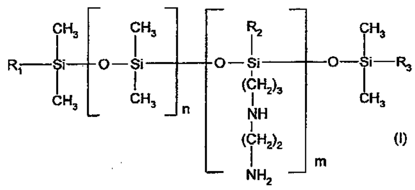

(I)

in which formula (I):

   m and n are numbers such that the sum (n + m) ranges from 1 to 1 000,
   n denoting a number from 0 to 999 and m denoting a number from 1 to 1 000;
   $R_1$, $R_2$, $R_3$, which may be identical or different, represent a hydroxyl or $C_1$-$C_4$ alkoxy radical, at least one of the radicals $R_1$ to $R_3$ denoting an alkoxy radical;

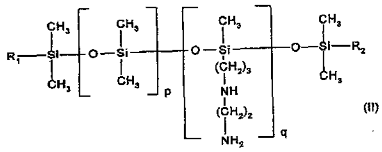

(II)

in which formula (II):

p and q are numbers such that the sum (p + q) ranges from 1 to 1 000,
p denoting a number from 0 to 999 and q denoting a number from 1 to 1 000;
$R_1$, $R_2$, which may be identical or different, represent a hydroxyl or $C_1$-$C_4$ alkoxy radical, at least one of the radicals $R_1$ to $R_2$ denoting an alkoxy radical.

2. Process according to Claim 1, **characterized in that** the $C_1$-$C_4$ alkoxy radical denotes the methoxy radical.

3. Process according to either of the preceding claims, **characterized in that**, for the aminosilicones of formula (I), the hydroxyl/alkoxy molar ratio is between 0.2:1 and 0.4:1.

4. Process according to either of Claims 1 and 2, **characterized in that**, for the aminosilicones of formula (II), the Hydroxyl/Alkoxy molar ratio is between 1:0.8 and 1:1.1.

5. Process according to any one of Claims 1 to 3, **characterized in that** the aminosilicone of formula (I) has a weight-average molecular mass ranging from 2 000 to 1 000 000.

6. Process according to any one of Claims 1 to 2 and 4, **characterized in that** the aminosilicone of formula (II) has a weight-average molecular mass ranging from 2 000 to 200 000.

7. Process according to any one of the preceding claims, **characterized in that** the pH of the pre-treatment or post-treatment composition comprising at least one aminosilicone is preferably between 2 and 10.

8. Process according to any one of the preceding claims, **characterized in that** the concentration of non-microemulsified aminosilicones of formula (I) or (II) in the pre-treatment or post-treatment composition is between 0.05 and 10% by weight relative to the total weight of this composition.

9. Process according to any one of the preceding claims, **characterized in that** the pre-treatment or post-treatment composition is left to act on the hair for a period of between 1 and 60 minutes.

10. Process according to any one of the preceding claims, **characterized in that** the pre-treatment or post-treatment composition also comprises additives chosen from vitamins and derivatives thereof such as vitamin E, vitamin E acetate, vitamin C and its esters, the B vitamins, vitamin A alcohol or retinol, vitamin A acid or retinoic acid and its derivatives, provitamins, vitamin A palmitate, niacinamide, ergocalciferol, antioxidants, essential oils, wetting agents, silicone or non-silicone sunscreens, preserving agents, sequestering agents, pearlescent agents, pigments, moisturizers, antidandruff agents, anti-seborrhoeic agents, plasticizers, hydroxy acids, electrolytes, solvents and fragrances.

11. Kit for the permanent reshaping of keratin fibres, one of the compartments containing a pre-treatment and/or post-treatment cosmetic composition comprising at least one aminosilicone as defined in any one of Claims 1 to 8.

12. Use of a pre-treatment and/or post-treatment cosmetic composition comprising, in a cosmetically acceptable vehicle, at least one aminosilicone as defined in any one of Claims 1 to 8, for the permanent reshaping of keratin fibres.

**Patentansprüche**

1. Verfahren für die dauerhafte Verformung von Keratinfasern und insbesondere Haaren, das zumindest die folgenden Schritte umfasst, bestehend aus:

(i) Aufbringen einer reduzierenden Zusammensetzung auf die Keratinfasern;
(ii) Durchführung der Oxidation der Keratinfasern,

**dadurch gekennzeichnet, dass** es ferner umfasst, vor Schritt (i) und/oder nach Schritt (ii) auf die Keratinfasern eine kosmetische Zusammensetzung zur Vorbehandlung und/oder zur Nachbehandlung aufzubringen, die in einem kosmetisch akzeptablen Träger mindestens ein aminiertes Silicon in einer nicht mikroemulgierten Form der folgenden Formeln (I) oder (II) enthält:

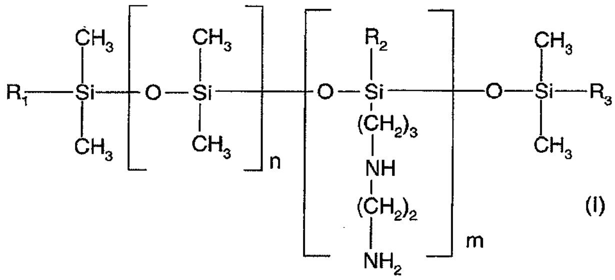

(I)

in der Formel (I):

m und n bedeuten Zahlen, die so gewählt sind, dass die Summe (n + m) im Bereich von 1 bis 1000 liegt,
n ist eine Zahl im Bereich von 0 bis 999 und m ist eine Zahl im Bereich von 1 bis 1000,
$R_1$, $R_2$ und $R_3$, die gleich oder verschieden sind, bedeuten eine Hydroxygruppe oder eine $C_{1-4}$-Alkoxygruppe, wobei mindestens eine der Gruppen $R_1$ bis $R_3$ eine Alkoxygruppe bedeutet;

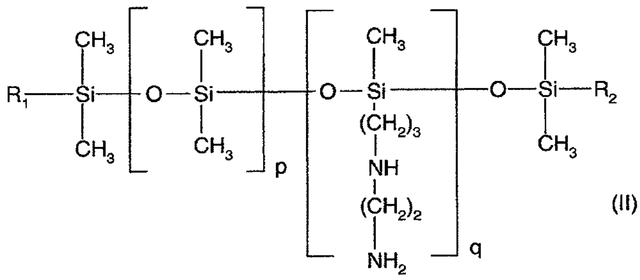

(II)

in der Formel (II):

p und q bedeuten Zahlen, die so gewählt sind, dass die Summe (p + q) im Bereich von 1 bis 1000 liegt,
p ist eine Zahl im Bereich von 0 bis 999 und q ist eine Zahl im Bereich von 1 bis 1000,
$R_1$ und $R_2$, die verschieden sind, bedeuten eine Hydroxygruppe oder eine $C_{1-4}$-Alkoxygruppe, wobei mindestens eine der Gruppen $R_1$ bis $R_2$ eine Alkoxygruppe bedeutet.

**2.** Verfahren nach Anspruch 1, **dadurch gekennzeichnet, dass** die $C_{1-4}$-Alkoxygruppe die Methoxygruppe bedeutet.

**3.** Verfahren nach einem der vorhergehenden Ansprüche, **dadurch gekennzeichnet, dass** für die aminierten Silicone der Formel (I) das Molverhältnis Hydroxy/Alkoxy im Bereich von 0,2:1 bis 0,4:1 liegt.

**4.** Verfahren nach einem der Ansprüche 1 bis 2, **dadurch gekennzeichnet, dass** für die aminierten Silicone der Formel (II) das Molverhältnis Hydroxy/Alkoxy im Bereich von 1:0,8 bis 1:1,1 liegt.

**5.** Verfahren nach einem der Ansprüche 1 bis 3, **dadurch gekennzeichnet, dass** das aminierte Silicon der Formel (I) eine gewichtmittlere Molmasse im Bereich von 2.000 bis 1.000.000 aufweist.

**6.** Verfahren nach einem der Ansprüche 1 bis 2 und 4, **dadurch gekennzeichnet, dass** das aminierte Silicon der Formel (II) eine gewichtmittlere Molmasse im Bereich von 2.000 bis 200.000 aufweist.

7. Verfahren nach einem der vorhergehenden Ansprüche, **dadurch gekennzeichnet, dass** der pH-Wert der Zusammensetzung für die Vorbehandlung oder für die Nachbehandlung, die mindestens ein aminiertes Silicon enthält, im Bereich von vorzugsweise 2 bis 10 liegt.

8. Verfahren nach einem der vorhergehenden Ansprüche, **dadurch gekennzeichnet, dass** die Konzentration der nicht mikroemulgierten aminierten Silicone der Formel (I) oder (II) in der Zusammensetzung für die Vorbehandlung oder für die Nachbehandlung im Bereich von 0,05 bis 10 Gew.-%, bezogen auf das Gesamtgewicht der Zusammensetzung, liegt.

9. Verfahren nach einem der vorhergehenden Ansprüche, **dadurch gekennzeichnet, dass** die Zusammensetzung für die Vorbehandlung oder für die Nachbehandlung während einer Zeitdauer im Bereich von 1 bis 60 Minuten auf die Haare einwirken gelassen wird.

10. Verfahren nach einem der vorhergehenden Ansprüche, **dadurch gekennzeichnet, dass** die Zusammensetzung für die Vorbehandlung oder für die Nachbehandlung ferner Additive enthält, die unter den Vitaminen und ihren Derivaten, wie Vitamin E, Vitamin-E-actetat, Vitamin C und seinen Estern, B-Vitaminen, Vitamin-A-alkohol oder Retinol, Vitamin-A-säure oder Retinsäure und ihren Derivaten, Provitaminen, Vitamin-A-palmitat, Niacinamid, Ergocaliciferol, Antioxidantien, etherischen Ölen, Feuchthaltemitteln, Sonnenschutzfiltern, die siliconiert oder nicht siliconiert sind, Konservierungsmittel, Maskierungsmitteln, Perlglanzstoffen, Pigmenten, Hydratisierungsmitteln, Wirkstoffen gegen Schuppen, Wirkstoffen gegen Seborrhöe, Weichmachern, Hydroxysäuren, Elektrolyten, Lösemitteln und Parfums ausgewählt sind.

11. Kit für die dauerhafte Verformung von menschlichen Keratinfasern, wobei eine Abteilung eine Zusammensetzung für die Vorbehandlung und/oder für die Nachbehandlung enthält, die mindestens ein aminiertes Silicon enthält, wie es in einem der Ansprüche 1 bis 8 definiert ist.

12. Verwendung einer kosmetischen Zusammensetzung für die Vorbehandlung und/oder für die Nachbehandlung, die in einem kosmetisch akzeptablen Medium mindestens ein aminiertes Silicon enthält, wie es in einem der Ansprüche 1 bis 8 definiert ist, für die dauerhafte Verformung von Keratinfasern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- JP H2250814 B
- JP H9151120 B
- GB 2141454 A
- EP 354835 A
- EP 368763 A
- EP A A
- EP 432000 A
- EP 465342 A
- EP 514282 A
- FR 2679448 A